(19)

Europäisches
Patentamt

European
Patent Office

Office européen
des brevets

(11) **EP 2 313 001 B1**

(12) **EUROPEAN PATENT SPECIFICATION**

(45) Date of publication and mention
of the grant of the patent:
**01.02.2012 Bulletin 2012/05**

(51) Int Cl.:
**A61B 5/053** $^{(2006.01)}$

(86) International application number:
**PCT/EE2009/000007**

(21) Application number: **09745476.3**

(22) Date of filing: **12.05.2009**

(87) International publication number:
**WO 2009/138093 (19.11.2009 Gazette 2009/47)**

(54) **METHOD AND DEVICE USING SHORTENED SQUARE WAVE WAVEFORMS IN SYNCHRONOUS SIGNAL PROCESSING**

VERFAHREN UND VORRICHTUNG MIT GEKÜRZTEN QUADRATISCHEN WELLENFORMEN BEI DER SYNCHRONEN SIGNALAUFBEREITUNG

PROCEDE ET DISPOSITIF UTILISANT DES FORMES D'ONDES CARREES RACCOURCIES DANS UN TRAITEMENT DE SIGNAUX SYNCHRONES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **12.05.2008 US 52467 P**

(43) Date of publication of application:
**27.04.2011 Bulletin 2011/17**

(73) Proprietor: **Tallinn University Of Technology**
**19086 Tallinn (EE)**

(72) Inventors:
• **ANNUS, Paul**
**EE-10146 Tallinn (EE)**
• **MIN, Mart**
**EE-13424 Tallinn (EE)**
• **OJARAND, Jaan**
**EE-10919 Tallinn (EE)**

(56) References cited:
**US-A1- 2006 100 539**

• **MART MIN ET AL: "Improvement of Lock-in Electrical Bio-Impedance Analyzer for Implantable Medical Devices" IEEE TRANSACTIONS ON INSTRUMENTATION AND MEASUREMENT, IEEE SERVICE CENTER, PISCATAWAY, NJ, US, vol. 56, no. 3, 1 June 2007 (2007-06-01), pages 968-974, XP011181321 ISSN: 0018-9456**

**Description**

TECHNICAL FIELD

The invention relates to methods of synchronous signal processing using shortened square wave waveforms. The method is particularly suitable for impedance measurements, e.g., of bioobjects such as tissues, organs, muscles, etc.

BACKGROUND ART

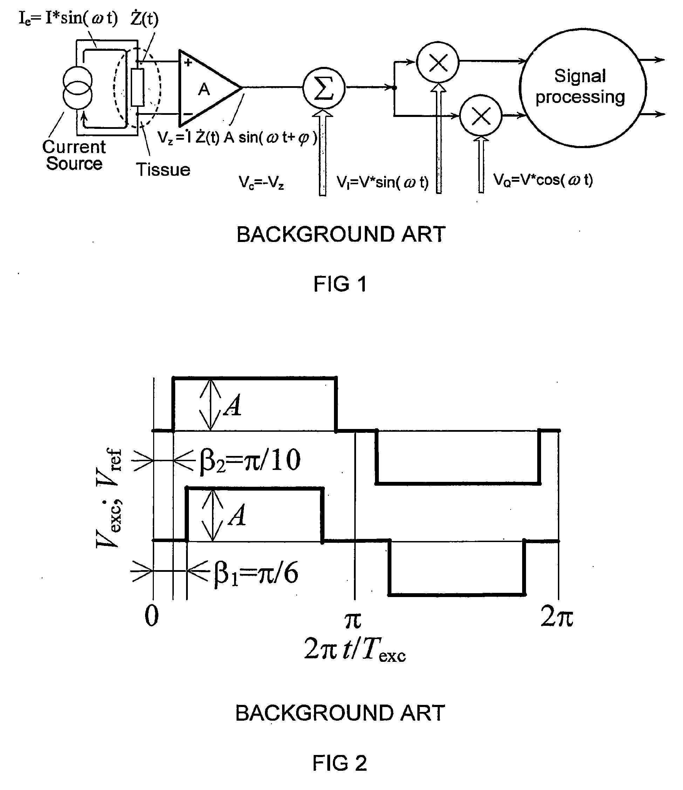

Impedance measurements are widely used for characterizing parameters of materials and substances, tissue parameters in biology and medicine, and even cell cultures [1]. When characterizing biological and medical parameters of tissue segments, electrical impedance is often called electrical bioimpedance (EBI) [2]. It could be measured at several different frequencies (multifrequency measurements), and sometimes also from multiple locations (multisite measurements) in order to derive multidimensional picture of tissue parameters under examination. EBI (in many cases together with visual inspection, temperature measurements etc.) gives valuable information, useful in formulating a diagnosis and providing proper medical care to the patient. It can be used for monitoring human heart functionality in pacemakers, during surgery, or for restoring normal blood flow after surgery. These measurements are usually conducted using synchronous signal processing. Same method enables measurement of low-level signals with lock-in amplifiers, and is used in different network analyzers. Classically sinusoidal excitation is used, and Fast Fourier Transformation (FFT) or similar is used for spectral separation. It enables determination of magnitude and phase of the response signal compared to the excitation signal, and gives relatively good results, depending on the quality of the excitation signal and signal processing algorithms. Typical signal chain for synchronous measurements is shown on Fig. 1, in which sine wave signals are used classically. Summing stage could be omitted, however in some circumstances compensation could improve resolution of the result. For out of the body applications size and energy consumption are not necessarily important, even though it is generally desirable to keep the energy consumption at minimum. Situation changes dramatically in case of implantable devices, such as pacemakers. Both analog circuitry and digital signal processing tend to consume a lot of energy. Also, size of the device should be kept as small as possible.

Instead of the sinusoidal signals, other waveforms can be used, provided that correlation between measurement results with sinusoidal signals can be shown with acceptable accuracy and repeatability. In wearable and implantable devices square wave excitation [3] can be used mainly because it is energy efficient, easy to generate, and easy to process. Substantial energy savings can be achieved at higher level of reliability (corresponds to reduced number of components). However, higher harmonics of non-sinusoidal waveforms cause errors, which need to be dealt with, or ideally suppressed [4]. Well-known solution is band pass filtering of the excitations signal [5]. Alternatively good results can be achieved by using different piecewise continuous approximations of sinusoidal signals [6]. Drawback associated with later approach is that harmonics content is very sensitive to level accuracy, and usually needs adjustments. Random and pseudo-random binary sequences are investigated, and maximum length sequences (MLS) are reported to give good results for high-speed impedance measurement of small particles in microfluidic cytometer [7]. Unfortunately it also involves rather complex signal processing at later stage including fast M-sequence transform (FMT), as well as FFT, before impedance information can be determined

Systematic errors introduced by higher harmonics of simple square wave signals can be drastically reduced by modifying the rectangular waveforms [8]. In case of shortening each rectangular half period of the excitation and reference signals by 30° and 18° correspondingly by introducing a section with zero amplitude at each end of the half period of the signals (see Fig. 2) the errors from higher odd harmonics can be reduced more than order of magnitude in comparison with regular rectangular waves.

Spectra of these signals can be expressed as the Fourier series of odd harmonics:

$$F(\omega t) = \frac{4A}{\pi}\left[\frac{\cos\beta}{1}\sin\omega t + \frac{\cos 3\beta}{3}\sin 3\omega t + \ldots\right] =$$

$$= \frac{4A}{\pi}\left[\sum_{i=1}^{\infty}\frac{\cos(2i-1)\beta}{2i-1}\sin(2i-1)\omega t\right] \qquad (1)$$

where A is the amplitude of the rectangular signal. In order to remove 3rd and 5th harmonics from the rectangular signal (as they cause the most significant errors) following simple conditions are valid for choosing the zero value intervals $\beta$:

for the 3rd harmonic $3 \cdot \beta = \pi/2$ must be fulfilled, which means that $\beta = 30°$

for the 5th harmonic $5 \cdot \beta = \pi/2$ must be fulfilled, which shows that $\beta = 18°$.

Synchronous demodulation is sensitive only to higher harmonics, which are existing simultaneously in both, the excitation and reference signals, such as the 7th, 11th, 13the, 17th, 19th, 23rd, 29th, and 31th in case of 30°/18° shortened signals.

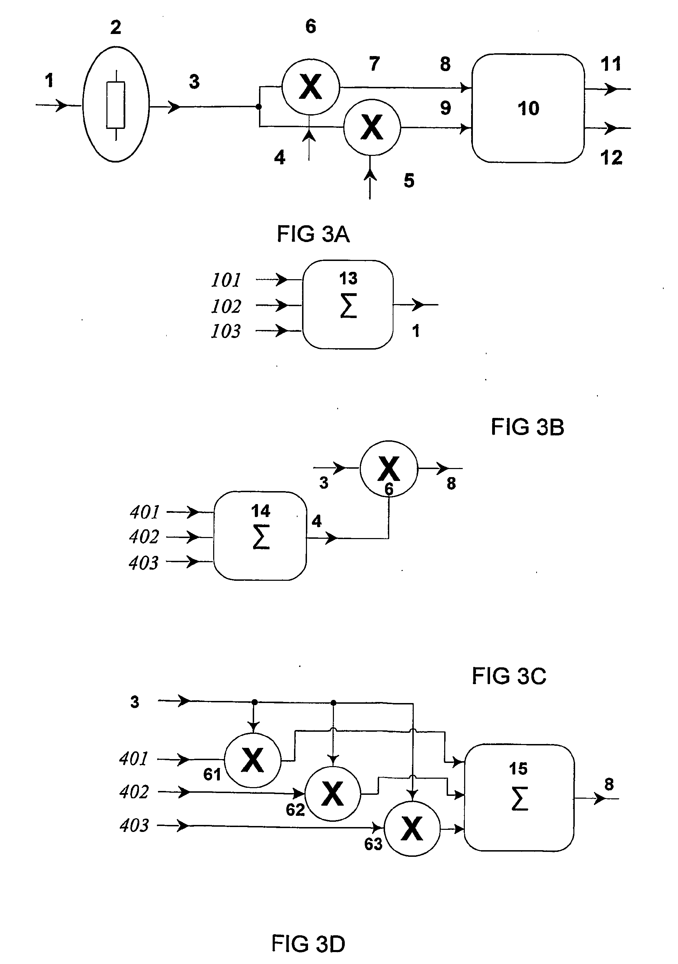

In some cases, these simultaneously existing higher harmonics have negligible effect on measurement accuracy. Impact of higher harmonics of the excitation and reference signals to the multiplication result in case of 30°/18° shortened signals can be seen in Fig. 3A. As a result, impact of the 5th, 9th, 15th, 21st, 25th, and 27th higher harmonics is absent. Fig. 3B is introduced for comparison with using of simple square waves.

However, there is a need for for higher accuracy than the prior art can offer. For example, the impact of the 7th harmonic has remained too substantial for providing precision measurements.

SUMMARY OF THE INVENTION

The objective of the invention is a method for impedance measurements comprising introducing a first modified rectangular signal into an object such as bioobject, e.g., a tissue, receiving a response signal from said object, introducing said response signal and a second modified rectangular signal into a synchronous detector, whereas either said first modified rectangular signal or said second modified rectangular signal, or both signals are so modified as to remove certain higher harmonics from the signals.

BRIEF DESCRIPTION OF THE DRAWINGS

Fig. 1 is a typical signal chain for synchronous measurements of impedance, where sinusoidal signals are traditionally used;

Fig. 2 depicts modified rectangular wave waveforms to be used in a set up of Fig 1 instead of sinusoidal signals, wherein the rectangular half periods of rectangular wave waveforms are shortened at each end by 18° and 30° respectively;

Fig. 3A depicts the system where the invented method can be used. Fig 3B illustrates generating the excitation signal according to the invention. Fig. 3C illustrates generating a reference signal according to the invention. Fig. 3D illustrates alternative set up for generating reference signal.

Fig. 4 comparatively show the higher harmonics present in different waveforms at the exit of the synchronous detector: 18°/30° shortened signal pair of Fig. 2 (white columns), an ordinary 50/50 duty cycle rectangular wave (dotted lines) and for signals shown in Figs 7A and 7B (black columns)

Fig. 5 depicts a modified rectangular wave signal, generated by summing three modified rectangular wave waveforms, shortened by 18°, 30° and 42° respectively (Fig 5A) and resulting spectral composition (Fig 5B);

Fig. 6 depicts a spectral view of modified rectangular wave waveforms, generated by summing three modified rectangular signals with adding coefficients 1, -0.5 and 0.5 (Fig 6A) and with coefficients 1, 1, 1 (Fig 6B);

Fig. 7 depicts a half periods of waveforms with adding coefficients 1, -0.5 and 0.5 (Fig 7A) and with coefficients 1, 1 and 1 (Fig 7B);

DETAILED DESCRIPTION OF THE INVENTIONS

The invention is now described with references to enclosed figures.

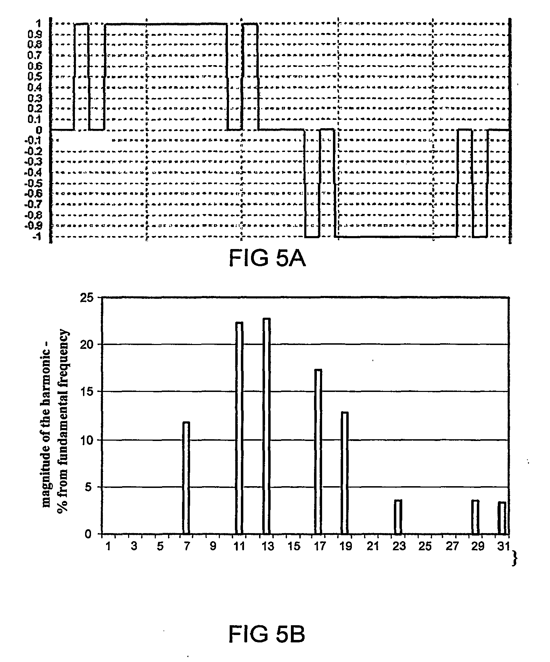

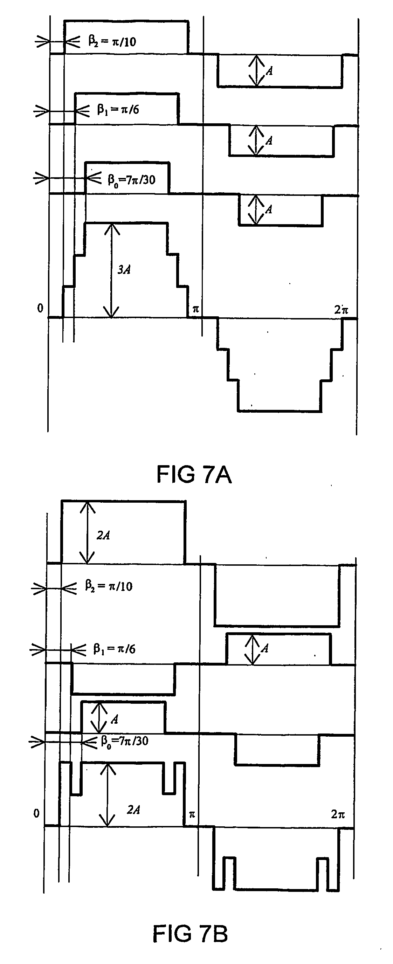

Fig. 3 depicts the principal set up where the invented method can be used. An excitation signal 1 is introduced into an object 2 such as bioobject, for example, a tissue. The excitation signal 2 can be any periodic signal with known frequency and harmonic content, such as sinusoidal signal, triangular signal, a rectangular signal that is modified as known from the art, e.g., as shown in FIG 1B, or any of the modified rectangular signals according to this invention (see Figs 5 and 7).

A response signal 3, corresponding to the excitation signal 1 is then received from the object 2 and introduced into synchronous detecting means 6, such as a synchronous detector. Synchronous detector 6 is used, e.g., for inphase signal and detector 7 for quadrature channel. A reference signal 4 (for inphase channel; reference signal 5 for quadrature channel) is also introduced into the synchronous detector. The reference signal is a modified rectangular signal according to this invention. "Modified rectangular signal" can be best described comparing to traditional rectangular signal. The traditional rectangular signal has amplitude A and period T so that the signal has value A during one of the half periods T/2 and value -A during the other half period T/2. Such signal has harmonic content represented by equation (1) above in the case, where the zero state is absent ($\beta = 0°$). The modified rectangular signal also has period T and amplitude A. However, each half period of the modified rectangular signal comprises zero amplitude segments ($\beta \neq 0°$), i.e., segments during which the value of the signal is zero. Such signal has a harmonic content improved compared to traditional rectangular signal in that at least one higher harmonic is at least partially suppressed.

For example, in known art, using signals as shown in Fig 2 the suppression of the impact of the 7th harmonic is roughly 5dB compared to square wave, but it is still remains high. However, it turns out that signals could be considerably improved without almost any added complexity in their generating. Such signal has zero value during first 18° segment, value A (e.g., unit value 1) during following 12° segment, zero value during the yet following 12° segment, value A during the next 96° segment, then zero value for 12° segment, value A for 12° segment and zero value during the last 18° segment of the half period T/2. The second half period is symmetrical to the first half period, having value -A instead of value A.

Such the signal can be easily generated by summing up three signals - the signals having the same shape as the signals Vref and Vexc shown in Fig. 2, and a third signal that is shortened similarly by 42° at the beginning and end of each half period. It appears that when summing 18° shortened signal with + sign, 30° shortened signal with - sign, and 42° shortened signal with + sign, the new waveform (Fig 5A) is free from both the 3rd and 5th harmonics. Resulting spectrum of such signal can be seen in Fig. 5B.

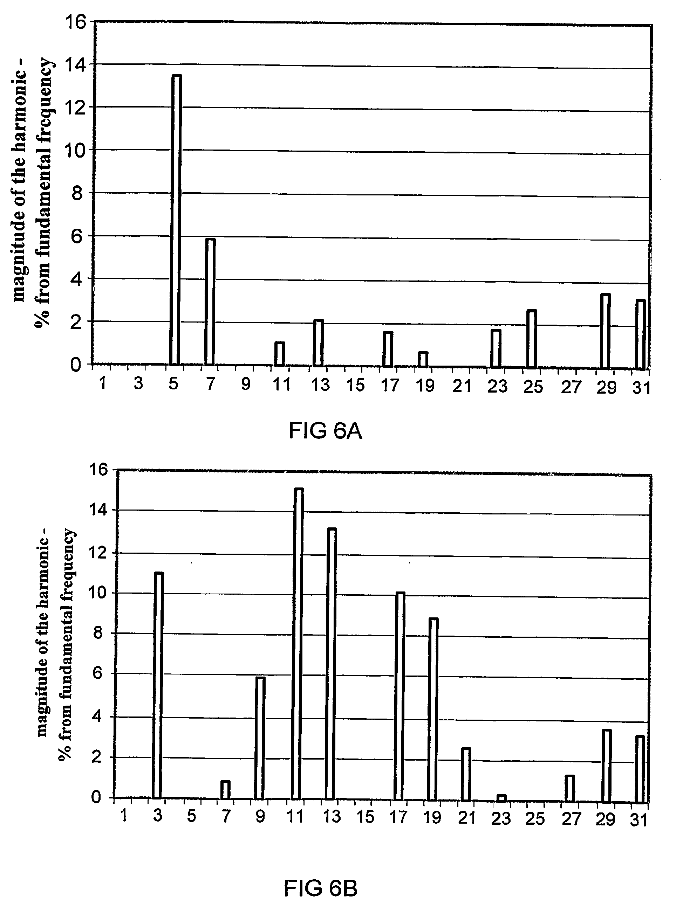

Even 7th harmonic has been somewhat reduced compared to the traditional square wave, however, the higher harmonics on the other hand are relatively high. The harmonic content of the signal can be further improved with changing the addition coefficients from 1, -1, 1 to 1, - 0,5, 0,5. Resulting spectrum can be seen in Fig. 6A, and waveform on Fig. 7A. Appearing 3rd and 9th harmonics could pose a problem, however in case of suitably chosen signal pair it is possible to eliminate them from the multiplication result. Good candidate for such a pairing signal is sum of 18°, 30° and 42° shortened signals with +1 coefficients. Resulting spectrum can be seen on Fig. 6B, and waveform on Fig. 6B. In order to show the difference two different multiplication results can be seen on Fig. 7: multiplication result of 30°/18° shortened signal pair compared to multiplication result of last two discussed signals.

Though the impact of the 7th harmonic is still present, the role of it has been reduced considerably (about 30 times, as it is discussed in example 1).

EXAMPLES

EXAMPLE 1

According to the first embodiment of the invention, the excitation signal 1 is a first modified rectangular signal, having improved harmonic content. Such excitation signal 1 is formed by adding three subsignals 101, 102 and 103 by summing means 13 (see Fig 3A). The subsignals and corresponding excitation signal 1 are shown in Fig. 7A and the spectrum of this signal is shown in Fig. 6A.

In this embodiment, similarly, the reference signal 4 to be introduced into synchronous detector 6 is generated by adding three subsignals 401, 402 and 403 by summing means 14 (see Fig 3C). The subsignals and corresponding reference signal is shown in Fig 7B and the spectrum of this signal is depicted in Fig 6B.

Even though the signal depicted in Fig. 7A is more preferred as excitation signal as it has higher excitation energy, in principle, also the signal shown in Fig. 7B used as the excitation signal and signal shown in Fig. 7A as the reference signal.

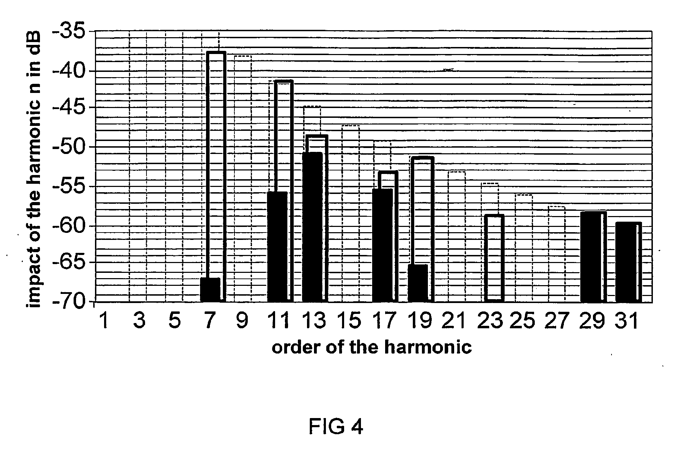

Impact of the higher harmonics is characterized by the spectrum of their multiplication product in Fig. 4. The role of the 7th harmonic is reduced 29 dB (from -38dB to -67 dB) or about 30 times compared to prior art. Also the role of 11th harmonic is reduced by 14 dB (near to 5 times). The role of 19th harmonic is reduced by 13 dB (about 4.5 times) and the role of 23rd harmonic is suppressed to negligible (lower than -70dB).

Alternatively, a set up according to Fig. 3D can be used for generating the reference signal. Instead of first summing up the subsignals 401 to 403, first the subsignals are introduced into synchronous detectors 61 to 63 respectively, and then the resulting signals are summed in summing means 15.

EXAMPLE 2 According to the second embodiment of the invention, the excitation signal is a rectangular signal, or alternatively, a modified rectangular signal know from the art. Our invention improves the measurement results considerably also in the case when one of the used two signals is a traditional rectangular one, but the other is modified according to the embodiment of our invention. In this case the most preferable modified signal is given in FIG. 5A, the spectrum of which does not contain the 3rd and the 5th higher harmonics. As a result, the impact of these higher harmonics is absent.

EXAMPLE 3 According to the third embodiment, a sinusoidal excitation signal is used. Our invention improves the signal-to-noise ratio and gives more exact measurement results even then, when the excitation signal is a traditional sine wave. The embodiment of improvement is based on the fact that zero value state of the reference signal impedes both the response signal and accompanied noise. When the noise level is high then the segment of impeded noise can be higher than the segment of sine wave. The most reasonable modified signal is a simple modification of the signal shown in Fig 2 with the zero state segment is not 18° or 30° but preferably 22.5°, which value 360°/16 is very near to the mathematical optimum is 21.4°

SIGNAL PROCESSING AND GENERATION

Signal processing using shortened square wave pulses in real measurement is straightforward. In case of 30°/ 18° shortened signals one of them, for example 18° shortened signal, is used for excitation current generation, while other, in this case 30° shortened signal, for multiplication. In reality it is enough to just cumulatively add samples from 30° to 150°, and sub-tract samples from 210° to 330° to get real part of the impedance, and add samples from 120° to 240°, and subtract samples from 0° to 60° and 300° to 360° to get imaginary part of the impedance under examination. If it is done for integer number of signal periods signal to noise ratio could be further improved. Undersampling is also easily accomplished, if needed. In simplest from 61/n*60 ratio should be maintained between sampling and signal forming clocks.

There is very little added complexity with discussed summed signals. In case of real part calculation samples between 30° to 42°, 138° to 150°, 210° to 222°, and 318° to 330° should be divided by two before summing, which in digital terms means simple shifting. For imaginary part same is valid, just location of the samples to be divided are shifted 90° from previously discussed. Signal forming for excitation is similarly simple. It is worth noting here that compared to piecewise continuous approximations of sinusoidal signals (known in the art), the method according to the invention using equal levels is much more feasible in digital domain of signal generation.

Replacing strictly sinusoidal signals with pulse waves in bioimpedance measurement device leads to measurement errors caused by higher harmonics. While it is not possible to eliminate these errors, usage of different carefully selected shortened pulse waves for excitation and for demodulation can minimize their impact to measurement results. Clear advantage in terms of simplicity of realization, together with very low current consumption, can be achieved due to the nature of such pulses. Just selecting correct samples, possibly shifting them, and then adding together can accomplish numerical synchronous demodulation. New, still simple, waveforms can be introduced by performing simple binary weighted additions on basic three level waveforms. It improves spectral purity of the signal processing, and reduces errors introduced by higher harmonics. The 3rd, 5th and 9th harmonics are still missing from the result, and impact of the 7th harmonic is reduced by almost 30dB. Contemporary technology is well suited for practical realization of small form factor and energy efficient measurement devices based on introduced signals. Results can be used in clinical experiments or ultimately for improving wearable and implantable devices using EBI as vital source of information.

REFERENCES

[1] Cheung K, Gawad S, Renaud P (2005) Impedance Spectroscopy Flow Cytometry: On-Chip Label-Free Cell Differentiation. Wiley-Liss, Cytometry Part A 65A, 124-132.

[2] Grimnes S, Martinsen Ø G (2000) Bioimpedance and Bioelectricity Basics. Academic Press, London.

[3] Webster J G (Ed.) (1995) Design of Cardiac Pacemakers. IEEE Press, New York.

[4] Meade M L (1989) Lock-in Amplifiers: Principles and Applications. Peregrinus, London.

[5] Yúfera A, Leger G, Rodriguez-Villegas E O, Muñoz J M, Rueda Ivorra A A, Gomez R, Noguera N, Aguiló J (2002) An integrated circuit for tissue impedance measure, in Proc. of the IEEE EMBS Special Topic Conference on Microtechnologies in Medicine and Biology, 2002, pp. 88-93.

[6] Min M, Parve T, Kukk V, Kuhlberg A (2002) An Implantable Analyzer of Bio-Impedance Dynamics: Mixed Signal Approach. IEEE Transactions on Instrumentation and Measurement, Vol. 51, No. 4, August 2002, pp 674-678.

[7] Sun T, Holmes D, Gawad S, Green N, Morgan H (2007) High speed multi-frequency impedance analysis of single particles in a microfluidic cytometer using maximum length sequences. Royal Society of Chemistry, Lab Chip 7, 1034-1040.

[8] Min M, Parve T (2007) Improvement of Lock-in Electrical Bio- Impedance Analyzer for Implantable Medical Devices. IEEE Transactions on Instrumentation and Measurement, Vol. 56, No. 3, June 2007, pp 968-974.

Although this invention is described with respect to a set of aspects and embodiments, modifications thereto will be apparent to those skilled in the art. The foregoing description of the embodiments of the invention has been presented for the purposes of illustration and description. It is not intended to be exhaustive or to limit the invention to the precise form disclosed. Many modifications and variations are possible in light of this disclosure. It is intended that the scope of the invention be limited not by this detailed description, but rather by the claims appended hereto.

## Claims

1. A method for measuring an impedance of an object (2), the method comprising introducing a first periodic signal (1) into the object, receiving a response signal (3) from the object, introducing said response signal (3) and a second periodic signal (4) into a synchronous detector (6), wherein said first periodic signal (1) and said second periodic signal (4) have the same main frequency **characterized in that** generating at least one of said first periodic signal (1) and said second periodic signal (4) by summing at least a first and a second modified rectangular subsignals (101,102,103,401,402,403), at least one of said, modified rectangular subsignals comprising a rectangular half periods that are shortened by zero amplitude segments at each end of said subsignal, wherein the length of the zero amplitude segment is selected to suppress particular higher harmonics **in that** signal.

2. The method as in claims 1, wherein said first modified rectangular signal is generated by summing a first modified rectangular subsignal (101), a second modified rectangular subsignal (102), and a third rectangular subsignal (103), said first modified rectangular subsignal (101) comprising rectangular half periods that are shortened by first zero amplitude segments with the length of 18° at both ends of each rectangular half periods, said second modified rectangular subsignal (102) comprising rectangular half periods that are shortened by second zero amplitude segments with the length of 30° at both ends of each rectangular half period and said third modified rectangular subsignal (103) comprising rectangular half periods that are shortened by third zero amplitude segments with the length, of 42° at both ends of each rectangular half period, said signals being summed with coefficients 1, -0.5 and 0.5 respectively, and wherein said second modified rectangular signal is generated summing a fourth modified rectangular subsignal (401) a fifth modified rectangular subsignal (402), and a sixth rectangular subsignal (403), said fourth modified rectangular subsignal (401) comprising rectangular half periods that are shortened by a fourth zero amplitude segment with the length of 18° at both ends of each rectangular half periods, said fifth modified rectangular subsignal comprising rectangular half periods that are shortened by a fifth zero amplitude segment with the length of 30° at both ends of each rectangular half period and said sixth modified rectangular subsignal (403) comprising rectangular half periods that are shortened by a sixth zero amplitude segment with the length of 42° at both ends of each rectangular half periods, wherein said fourth, fifth and sixth subsignals are summed with coefficient 1.

## Patentansprüche

1. Verfahren zur Messung der Impedanz eines Objekts (2), das Verfahren umfasst die Einleitung des ersten periodischen Signals (1) in das Objekt, den Empfang eines Antwortsignals (3) von dem Objekt, die Einleitung des genannten Antwortsignals (3) und eines zweiten periodischen Signals (4) in einen synchronen Detektor (6), worin das genannte erste periodische Signal (1) und das genannte zweite periodische Signal (4) dieselbe Hauptfrequenz aufweisen, **dadurch gekennzeichnet, dass** beim Generieren von mindestens einem des genannten ersten periodischen Signals (1) und des genannten zweiten periodischen Signals (4) durch Summierung von mindestens die erste und die zweite modifizierten rechtwinkligen Untersignalen (101, 102, 103, 401, 402, 403) mindestens eines der genannten modifizierten rechtwinkligen Untersignale rechtwinklige Halbperioden umfasst, die an beiden Wenden des genannten Untersignals durch Nullamplitudensegmente gekürzt werden, worin die Länge des Nullamplitudensegments zwecks Unterdrückung von spezifischen Oberschwingungen in diesem Signal gewählt wird.

**2.** Verfahren nach Anspruch 1, worin das genannte erste modifizierte rechtwinklige Signal durch die Summierung des ersten modifizierten rechtwinkligen Untersignals (101), des zweiten modifizierten rechtwinkligen Untersignals (102) und des dritten modifizierten rechtwinkligen Untersignals (103) generiert wird, das genannte erste modifizierte rechtwinklige Untersignal (101) umfasst rechtwinklige Halbperioden, die durch erste Nullamplitudensegmente mit einer Länge von 18° an beiden Enden jeder rechtwinkligen Halbperiode gekürzt werden, das zweite modifizierte rechtwinklige Untersignal (102) umfasst rechtwinklige Halbperioden, die durch die zweite Nullamplitudensegmenten mit einer Länge von 30° an beiden Enden jeder rechtwinkligen Halbperiode gekürzt werden und das genannte dritte modifizierte rechtwinklige Untersignal (103) umfasst rechtwinklige Halbperioden, die durch dritte Nullamplitudensegmente mit einer Länge von 42° an beiden Enden jeder rechtwinkligen Halbperiode gekürzt werden, diese Signale werden mit den jeweiligen Koeffizienten 1, -0.5 und 0.5 summiert sein und worin das genannte zweite modifizierte rechtwinklige Signal durch die Summierung des vierten modifizierten rechtwinkligen Untersignals (401), des fünften modifizierten rechtwinkligen Untersignals (402) und des sechsten modifizierten rechtwinkligen Untersignals (403) generiert wird, das genannte vierte modifizierte rechtwinklige Untersignal (401) umfasst rechtwinklige Halbperioden, die durch das vierte Nullamplitudensegment mit einer Länge von 18° an beiden Enden jeder rechtwinkligen Halbperiode gekürzt werden, das fünfte modifizierte rechtwinklige Untersignal umfasst rechtwinklige Halbperioden, die durch das fünfte Nullamplitudensegment mit einer Länge von 30° an beiden Enden jeder rechtwinkligen Halbperiode gekürzt werden und das genannte sechste modifizierte rechtwinklige Untersignal (403) umfasst rechtwinklige Halbperioden, die durch das sechste Nullamplitudensegment mit einer Länge von 42° an beiden Enden jeder rechtwinkligen Halbperiode gekürzt werden, worin das genannte vierte, fünfte und sechste Untersignal mit dem Koeffizienten 1 summiert wird.

## Revendications

**1.** Une méthode pour mesurer l'impédance d'un objet (2), la méthode comprenant l'introduction d'un premier signal périodique (1) dans l'objet, la réception d'un signal de réponse (3) depuis l'objet, l'introduction dudit signal de réponse (3) et un second signal périodique (4) dans un détecteur synchrone (6), ou ledit premier signal périodique (1) et ledit second signal Périodique (4) ont la même fréquence principale, **caractérisée en ce que** la production d'au moins un dudit premier signal périodique (1) et dudit second signal périodique (4) est faite en additionnant au moins un premier et un second sous-signaux rectangulaires modifiés (101, 102, 103, 401, 402, 403), au moins l'un desdits sous-signaux rectangulaires modifiés comprenant des demi-périodes rectangulaires qui sont raccourcies par des segments d'amplitude zéro à chaque extrémité dudit sous-signal, où la longueur du segment d'amplitude zéro est choisie pour filtrer les hautes harmoniques dans ce signal.

**2.** La méthode comme dans la revendication 1, où le premier signal rectangulaire modifié est produit en additionnant un premier sous-signal rectangulaire modifié (101), un second sous-signal rectangulaire modifié (102), et un troisième sous-signal rectangulaire modifié (103), ledit premier sous-signal rectangulaire modifié (101) comprenant des demi-périodes rectangulaires qui sont raccourcies par des segments d'amplitude zéro avec la longueur de 18° à chaque extrémité de chaque demi-période rectangulaire, ledit second sous-signal rectangulaire modifié (102) comprenant des demi-périodes rectangulaires qui sont raccourcies par des segments d'amplitude zéro avec la longueur de 30° à chaque extrémité de chaque demi-période rectangulaire et ledit troisième sous-signal rectangulaire modifié (103) comprenant des demi-périodes rectangulaires qui sont raccourcies par des segments d'amplitude zéro avec la longueur de 42° à chaque extrémité de chaque demi-période rectangulaire, lesdits signaux étant additionnés avec les coefficients 1, -0.5 et 0.5 respectivement, et où le second signal rectangulaire modifié est produit en additionnant un quatrième sous-signal rectangulaire modifié (401), un cinquième sous-signal rectangulaire modifié (402), et un sixième sous-signal rectangulaire modifié (403), ledit quatrième sous-signal rectangulaire modifié (401) comprenant des demi-périodes rectangulaires qui sont raccourcies par un quatrième segment d'amplitude zéro avec une longueur de 18° à chaque extrémité de chaque demi-période rectangulaire, ledit cinquième sous-signal rectangulaire modifié comprenant des demi-périodes rectangulaires qui sont raccourcies par un cinquième segment d'amplitude zéro avec une longueur de 30° à chaque extrémité de chaque demi-période rectangulaire et ledit sixième sous-signal rectangulaire modifié (403) comprenant des demi-périodes rectangulaires qui sont raccourcies par un sixième segment d'amplitude zéro avec une longueur de 42° à chaque extrémité de chaque demi-période rectangulaire, où lesdits quatrième, cinquième et sixième sous-signaux sont additionnés avec un coefficient 1.

$I_e = I*\sin(\omega t)$   $\dot{Z}(t)$

$V_z = \dot{I} \dot{Z}(t) A \sin(\omega t + \varphi)$

Current
Source

Tissue

$V_c = -V_z$   $V_I = V*\sin(\omega t)$   $V_Q = V*\cos(\omega t)$

Signal
processing

BACKGROUND ART

FIG 1

$V_{exc}; V_{ref}$

$A$

$\beta_2 = \pi/10$

$A$

$\beta_1 = \pi/6$

$0$   $\pi$   $2\pi$

$2\pi\, t/T_{exc}$

BACKGROUND ART

FIG 2

FIG 3A

FIG 3B

FIG 3C

FIG 3D

FIG 4

FIG 5A

FIG 5B

FIG 6A

FIG 6B

FIG 7A

FIG 7B

13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Impedance Spectroscopy Flow Cytometry: On-Chip Label-Free Cell Differentiation. **CHEUNG K ; GAWAD S ; RENAUD P.** Cytometry. Wiley-Liss, 2005, vol. 65A, 124-132
- **GRIMNES S ; MARTINSEN Ø G.** Bioimpedance and Bioelectricity Basics. Academic Press, 2000
- Design of Cardiac Pacemakers. IEEE Press, 1995
- **MEADE M L.** Lock-in Amplifiers: Principles and Applications. Peregrinus, 1989
- **YÚFERA A ; LEGER G ; RODRIGUEZ-VILLEGAS E O ; MUÑOZ J M ; RUEDA IVORRA A A ; GOMEZ R ; NOGUERA N ; AGUILÓ J.** An integrated circuit for tissue impedance measure. *Proc. of the IEEE EMBS Special Topic Conference on Microtechnologies in Medicine and Biology,* 2002, 88-93

- **MIN M ; PARVE T ; KUKK V ; KUHLBERG A.** An Implantable Analyzer of Bio-Impedance Dynamics: Mixed Signal Approach. *IEEE Transactions on Instrumentation and Measurement,* August 2002, vol. 51 (4), 674-678
- High speed multi-frequency impedance analysis of single particles in a microfluidic cytometer using maximum length sequences. **SUN T ; HOLMES D ; GAWAD S ; GREEN N ; MORGAN H.** Lab Chip. Royal Society of Chemistry, 2007, vol. 7, 1034-1040
- **MIN M ; PARVE T.** Improvement of Lock-in Electrical Bio- Impedance Analyzer for Implantable Medical Devices. *IEEE Transactions on Instrumentation and Measurement,* June 2007, vol. 56 (3), 968-974